# EUROPEAN PATENT APPLICATION

(11) **EP 3 305 351 A1**
(43) Date of publication of application: **11.04.2018**
(21) Application number: 16803614.3
(22) Date of filing: 04.05.2016
(51) Int. Cl.: A61M 5/31, A61M 5/165

(54) **FILTERING NEEDLE PROVIDED WITH ROTATIONALLY CUTTABLE AND REMOVABLE INTAKE TUBE, AND FILTERING SYRINGE**

(30) Priority: 29.05.2015 KR 20150075622
(71) Applicant: Yuk, Young Su, Daejeon 34818 (KR)
(72) Inventor: Yuk, Young Su, Daejeon 34818 (KR)
(74) Representative: Patentanwälte Lambsdorff & Lange
(86) International application number: PCT/KR2016/004772
(87) International publication number: WO 2016/195259

(57) **Abstract**

Provided are a filtering needle, which has a rotationally cuttable and removable intake tube coupled thereto, and a filtering syringe using same. The present invention relates to a filtering needle, which overcomes the shortcoming of a conventional filtering syringe in which while the conventional filtering syringe performs a filtering intake and the discharge of an injectable solution using one needle, glass fragments, which do not flow deeply into the inner channel of a filter but remain between the needle and the filter, are introduced into a human body via the needle during an injection, the filtering needle being characterized in that while an intake part (20) provided with an intake tube, which is slightly larger than the needle, and capable of being spirally cut is placed over a needle part (10) and then rotationally removed therefrom, a connecting tube (24) inside the intake part and embedded in an inlet (16) comes out and simultaneously interlocks with a rotary plate plug (14) of the needle part, thereby closing the inlet (16) connected to the filter (15) and opening an outlet (17) connected to the needle (11). Owing to the above simple and sophisticated operation of soft components, the needle remains in the state of a bidirectionally open outflow tube, not a unidirectional conduit, even after completion of an intake. Accordingly, the status of blood detection can be confirmed by inserting the filtering needle into a human body and then slightly drawing in, as in the case of a general needle, and glass fragments filtered by the filter (15) are completely separated from the needle at this moment; thus, the filtering needle has the outstanding effect of preventing a filtered substance from being reinjected into the human body in comparison with conventional filtering syringes.

## Description

### [Technical Field]

The present invention relates to an injection needle provided with a filtering intake structure, and a filtering syringe adopting the same.

### [Background Art]

When a user opens a glass ampoule, which is frequently used as an injection container, by breaking a bottleneck, fine glass debris powder is generated. The debris powder that is not visible but is inserted into and mixed with an injection solution when the ampoule is opened may remain in a cylinder and an injection needle (hereinafter, referred to as a needle), and may be introduced into a human body during an injection process.

The glass debris powder having a diameter of largely up to 0.01 mm moves along blood vessels, is attached and settled in a specific position inside a body, and causes various diseases. Thus, to prevent this, a syringe having a suction liquid filtering function is actively used.

The related art document disclosed below relates to a patent in the field of filter syringes, of which applications and registrations have recently rapidly increased in Korea.

The overall technology trend of the filter syringe may be roughly classified into two kinds. One of them is a separation-type filter structure which is used in a state in which an intake tube having a filter embedded therein is separately fitted with an end of a needle, and prevents the debris powder from being introduced through the needle by sucking an injection liquid and then removing the intake tube. The other one of them is an internal flow-type filter structure which prevents the debris powder remaining in a suction-side filter from being inserted into a discharge tube, and is designed such that an internal passage of a filter unit is changed using a change in a direction of pressure differently applied during a suction process and a discharge process.

In particular, the latter flow-type filter structure has recently been spotlighted, and various derivative models have been patented and registered competitively. In a detailed description of an operation principle, a design structure in which a filter flow passage is changed according to a change in a direction of pressure applied to fluid inside a needle is commonly adopted.

### [Disclosure]

### [Technical Problem]

Advantages and disadvantages of the two filtering technologies described above may be clearly specified. First, a separation-type filter structure as a classic technology, in which a filter-embedded intake tube is separately connected to an end of a needle, will be described. In this technology, because a filter itself is removed after suction, a probability that debris powder remains inside the needle is fundamentally blocked. However, there is a concern that the needle may be polluted or a user may be injured while the intake tube connected to the end of the needle is separated and removed by hands. Above all, because the thickness or the length of the filter-embedded detachable intake tube is never small, it is difficult to apply the separation-type filter structure to a small ¾ ampoule or an ampoule having a narrow inlet.

Next, a flow-type filter structure having a structure in which one needle is in charge of suction and discharge of an injection liquid and a suction discharge passage is changed in a filter unit will be described.

A syringe to which this technology is applied is easy to use because there is not difference between the outer shape of the syringe and the outer shape of a general syringe not having a filter. However, because both suction and discharge should fundamentally be performed through one needle, when the debris powder is not deeply introduced into an inner passage of the filter with completely passing through the needle during the suction, and remains in the needle and an outer passage of the filter, the debris powder moves rearward toward the needle during the subsequent discharge, that is, an injection process, again, and thus, may be introduced into a human boy.

An object of the present invention is to comprehensively solve the disadvantages of the two filter syringes according to the related art.

In more detail, the specific technical improvements intended in the present invention are to successfully satisfy the following essential operating conditions.

The first operating condition is provided to maintain the inherent advantages of the conventional separating type suction pipe. A separate intake tube 21 completely separated from an injection needle is arranged together with a needle 11 so that an injection liquid is separately suctioned, and glass fragments are fundamentally prevented from being introduced into the injection needle.

As the second operating condition, in order to maintain the advantages of the syringe adopting the conventional flow-type filter structure, the intake tube 21 is shaped to have the thickness and the length minimally exceeding the size of the needle 11 so as to be almost completely compatible with the conventional ampoule, and is arranged to be adjacent to the needle (in fact, including the needle).

As the third operating condition, in the present invention, when the intake tube is removed before the syringe is inserted, the intake tube and the needle should be easily separated from each other without holding the needle such that the needle is not polluted. To this end, a suction part 20 including the intake tube is configured to entirely surround a needle part 10 including the needle.

An essential requirement for all the above operation conditions is that even after the intake tube separately arranged in the needle is separated, the injection liquid is prevented from being leaked to a side of the intake tube.

In addition to the above requirement, additional requirements are that a simple and intuitive manipulation method is presented in an overall manipulation process, safety of components performing complex effects such as separation and selective sealing is verified, and high operation reliability may be achieved even while an inexpensive conventional (disposable) injection supply material is mainly used.

### [Technical Solution]

The key point of the present invention to solve the above technical problems will be described above.

A structure is considered in which first, an injection liquid discharging needle 11 and an injection liquid suctioning intake tube 21 are separately provided, two tubes are arranged to be adjacent to each other, and the intake tube is deformed and partially cut to be removed from the needle such that only the needle is exposed alone to apply an injection after the injection liquid is suctioned.

In this process, the suction part 20 including the intake tube 21 may be manufactured of soft and nontoxic plastic material that can be bent or twisted. For example, soft and transparent polyethylene or polyvinyl chloride used for a syringe or caps of various sanitary apparatuses may be used.

That is, a material having balanced strength and flexibility is basically adopted. Further, the section thickness of a portion of the suction part 20, in which high strength is structurally required, is secured to some extent, but the portion of the suction part 20 is designed to be cut to complement stiffness. Further, a portion of the suction part 20, which should not be cut and in which high strength is not required structurally, is formed to be thinner than the other portion, and thus may be flexibly deformed.

When a general transparent and soft injection supply material is used, a portion of the suction part, which has a thickness of 1 mm or less, becomes very flexible and transparent, thereby assisting an intuitive operation of a user, and a portion of the suction part, which has a thickness of 2 mm or more, has strength and hardness enough to pierce an ampoule membrane formed of rubber while supporting the needle and internal flow cap structures well.

That is, because the section thickness of the suction part 20 is properly changed according to portions, and a cut groove 26 is cleverly and appropriately arranged in the suction part 20 in consideration of the change in the section thickness, the suction part 20 may perform an excessive operation greatly departing from an initial coupling state with an adjacent needle.

For reference, it is preferable that in a design process, a strong material enough to pierce a rubber membrane-type ampoule cap and even protect an end of the needle is applied to a tip of the intake tube 21.

Next, an additional technical conception that is as important as the above-described technical conception is a selective opening/closing effect for a filter 15 receiving an injection liquid from the suction part 20.

As in an embodiment of the drawing, the intake tube 21 and the filter 15 are connected to each other through one duct. When the injection liquid introduced into a cylinder through the duct is completely filled and the intake tube 21 is then removed such that the needle 11 is exposed, the duct and the filter 15 arranged in the duct are substantially opened. In this state, even when there is the separate injection liquid discharging needle 11, when a syringe piston is pressed, it is obvious that the injection liquid in the cylinder is simultaneously discharged to the filter 15 and the needle.

Therefore, in the present invention, the duct close to the filter 15 should be designed as a one-way permeable duct through which suction is possible and discharge is blocked before the duct is at least completely closed.

In many cases, the conventional technology frequently used in the above condition corresponds to an elastic valve. When a rubber membrane bent in one direction in a hinged scheme is located between the filter and the syringe cylinder, and is opened only on a side of the cylinder, the injection liquid is only suctioned through the filter but not discharged.

However, the above-described elastic valve has a serious problem so that the elastic valve should not be applied to an actual syringe.

Most injections are applied to blood vessels or muscles. At this time, a medical performer inserts a needle into a skin of a patient, and then pulls a plunger slightly to identify whether blood is introduced (a normal blood vessel is captured).

In this process, air may be introduced into the filter exposed and opened as a unidirectional valve is opened, and the introduced air enters the cylinder, and is then introduced into a human body through the needle as it is.

Thus, a general unidirectional valve structure is difficult to use in the field of a filtering syringe to which the present invention pertains. For reference, even in a syringe having a general flow filter structure (described above) currently on the market, in a process in which a needle is inserted into a skin, a plunger is pulled such that whether blood is introduced is identified, and an injection is applied again, glass fragments remaining in the filter may be attached to viscid blood and may then be injected into the human body.

Accordingly, in the present invention, a mechanical structure is conceived in which in a state in which an inlet hole on a side of the above-described filter 15 is initially and completely opened, suction is completed, and the inlet hole is then closed by an operation accompanied by a cutting and separating process of the suction portion 20.

Clearly, a disposable injection needle is not a large component for an easy mechanical design and but a precision component. In particular, the disposable injection needle, which is a product that should be mass-produced in consideration of production costs and hygiene, has an entirely simple and firm shape, and requires a sealed structure having high reliability.

In the present invention, an operational opening/closing mechanism providing convenience of operation while structural simplicity and sealing reliability that are close to those of the conventional disposable injection needle are maintained is added. The operational opening/closing mechanism corresponds to a rotating plate cap 14 interlocking with a cutting and separating operation of the suction part 20, and peripheral components thereof.

The rotating plate cap 14, which is a thin band-shaped soft member, may be formed of the same material as that of the suction part 20, or may be selectively formed of a silicone material for a human body, which has more excellent ductility and elasticity, to further improve a sealing force.

Referring to the accompanying drawings, when a connection tube 24 formed on an inner peripheral surface of the suction part 20 is inserted into the inlet hole 16 of the needle part 10 for a short time, and is then separated sideward, as the rotating plate cap 14 connected to the connection tube 24 is rotated in a rotating plate accommodating part 13 formed in a support 12 of the needle, the inlet hole 16 is closed.

In this process, a simple and certain operational cap is implemented in which characteristics of a soft and elastic material are fully utilized. Further, after the rotating plate cap 14 completely closes the filter, a coupling state of the rotating plate cap 14 and the connection tube 24 may be properly released to as needed to prevent fracture or rupture resulting from a cutting, rotating and separating movement of the entire suction part 20, which exceeds a predetermined range.

A detailed description supporting the above-described main technical conception in detail will be described below.

### [Effect]

According to the present invention, a filtering needle which completely blocks a probability that impurities such as debris powder are inserted into a human body is implemented. In particular, because the outer diameter of a separate suction tube is limited to about 3 to 6 mm that is close to the outer diameter of a needle, and the length of the suction tube slightly exceeds the length of the needle, a compact filtering needle that may be easily used with almost all ampoules for injections even without user's particular attention is implemented.

Further, while a suction part is removed after an injection liquid is suctioned, the needle is fundamentally prevented from being polluted. Thus, there is little inconvenience in use as compared to the conventional entry-level needle including only a cap and a needle. Further, the separated suction part is injected into the separated cap, and in this state, a needle part is fitted in the cap in turn, so that it is easy to discard the filtering needle after use.

Finally, all the above configurations may be implemented without greatly departing from materials, such as soft and transparent plastic, of the conventional injection supplies, so that a high-performance filtering needle may be manufactured with low production costs.

### [Description of Drawings]

FIG. 1 is a view entirely illustrating a filtering needle and a filtering syringe according to the present invention.
FIG. 2 is a view illustrating the filtering needle coupled to a syringe cylinder according to whether there is a cap.
FIG. 3 is an exploded view illustrating a main part of the filtering needle.
FIG. 4 is a perspective view and a sectional view illustrating an entire structure of the filtering needle.
FIG. 5 is a view illustrating a pre-prescription state in which after an injection liquid is completely suctioned, a suction part 20 slides, so that a needle is exposed.
FIGS. 6 and 7 are views illustrating a main operation structure of the filtering needle in detail.

### [Best Mode]

Embodiments of the present invention will be described with reference to the accompanying drawings to support the above-described technical solution of the present invention.

However, in the following embodiments, components expressed in specific terminologies and coupling structures thereof do not limit the technical spirit comprehensively inherent in the present invention.

First, a filtering needle presented throughout the drawings and the detailed description may be roughly divided into three parts including a needle part 10, a suction part 20, and a cap 30.

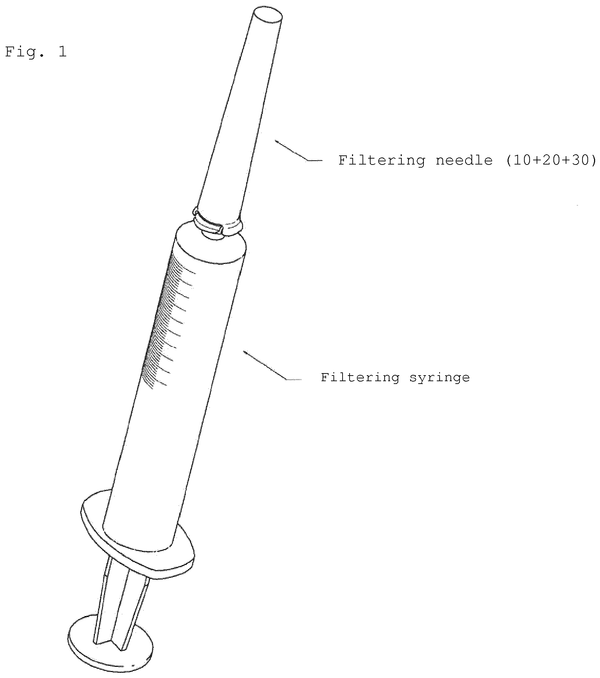

As illustrated in FIG. 1, the filtering needle including the three parts occupies most of the technical scope of the present invention, and may be itself one independent invention. The fact to notice in advance is that the filtering syringe on which the filtering needle is detachably or fixedly mounted is an invention belonging to the same category and is obviously included in the technical spirit of the present invention. That is, it should be interpreted that the filtering syringe having the filtering needle providing an independent completion function, which is an invention of a first group, naturally belongs to the scope of the right of the present invention in accordance with the common idea of the corresponding technical field.

For reference, in the present invention, the three parts have been defined as reference numerals 10, 20, and 30, respectively, and detailed structures or components of the three parts are defined to be included in reference numerals 11-19, 21-29, and 31-39, respectively.

For example, a component defined as one of reference numerals 11-19 is defined as a detailed configuration included in the needle part 10, and this definition scheme is applied even to the remaining two suction part 20 and the cap 30 as well.

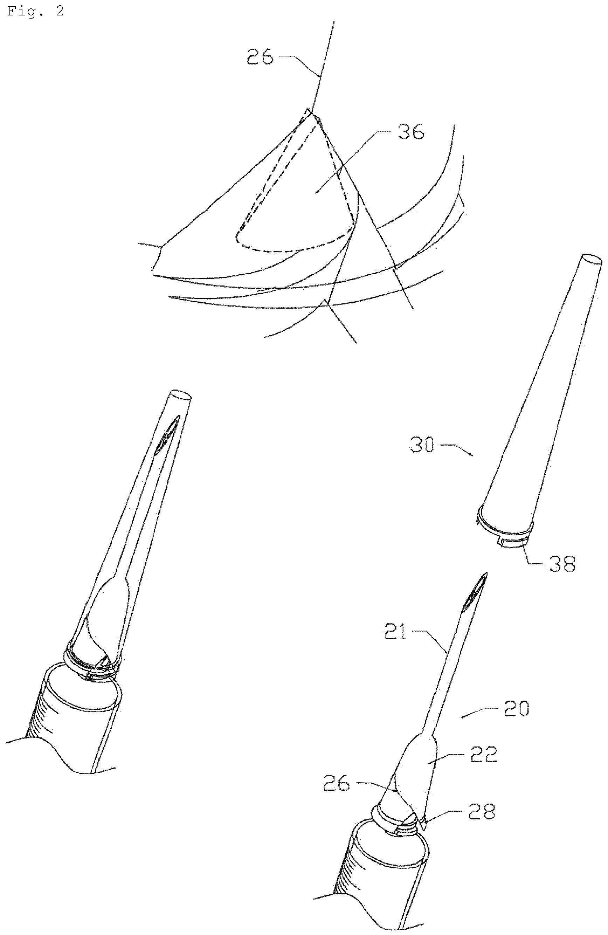

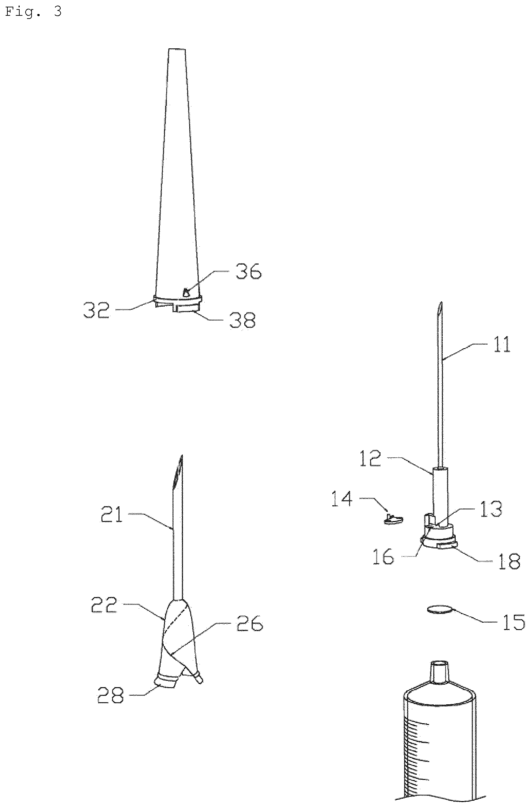

First, the entire shapes and structural characteristics of the three main parts will be described with reference to FIGS. 1 to 4.

The three parts entirely overlap and surround each other in a sequence of the needle part 10, the suction part 20, and the cap 30. In particular, the suction part 20 surrounds the needle part 10 to be matched with the shape of the needle part 10 well.

A vinyl wrapper (not illustrated) is opened, and the filtering needle may be inserted into an inlet of a syringe cylinder in an initial state itself in which the cap 30 is covered.

The outermost cap 30, which is a protection member that is fitted and coupled to the suction part 20, is configured to generate micro cutting, that is, a cutting notch, as a preliminary stage assisting in a cutting and rotating operation of the suction part using an opening operation of the cap as needed (for example, in the embodiment, it is described that a cutting blade 36 is provided).

The innermost needle part 10 has an upper portion to which a needle 11 is fixed, a lower portion in which a filter 15 is arranged, and a middle portion to which a rotating plate cap 14 having a shape of a fan-shaped plate is coupled. At this time, an initial state of the rotating plate cap 14 is a state in which an inlet hole 16 is opened and an outlet hole 17 is closed, that is, in a state in which an injection liquid is filtered and suctioned through the filter 15.

The suction part 20 formed to surround a circumference of the needle part 10 servers to supply the injection liquid introduced through the upper intake tube 21 to the filter 15. In detail, a portion formed from the intake tube 21 to a connection tube 24 on a lateral side of the needle 11 corresponds to one bendable passage, and is in a state in which the connection tube 24 is inserted into the inlet hole 16 in an initial assembling state. As will be described later, while the entire suction part 20 is cut, deformed, or separated, the connection tube 24 is bent and is separated from the inlet hole 16.

The suction part 20 has a streamlined conic shape having a section thickness of 1 mm to 5 mm, and may be injection-molded of soft and transparent polymer resin.

The intake tube 21 that is adjacent to the needle 11 is formed at an upper portion of the suction part 20, an extension 22 in which a cut groove 26 is formed is formed on a lower side of the intake tube 21, and a suction part handle 28 is formed near a lower edge below the extension 22.

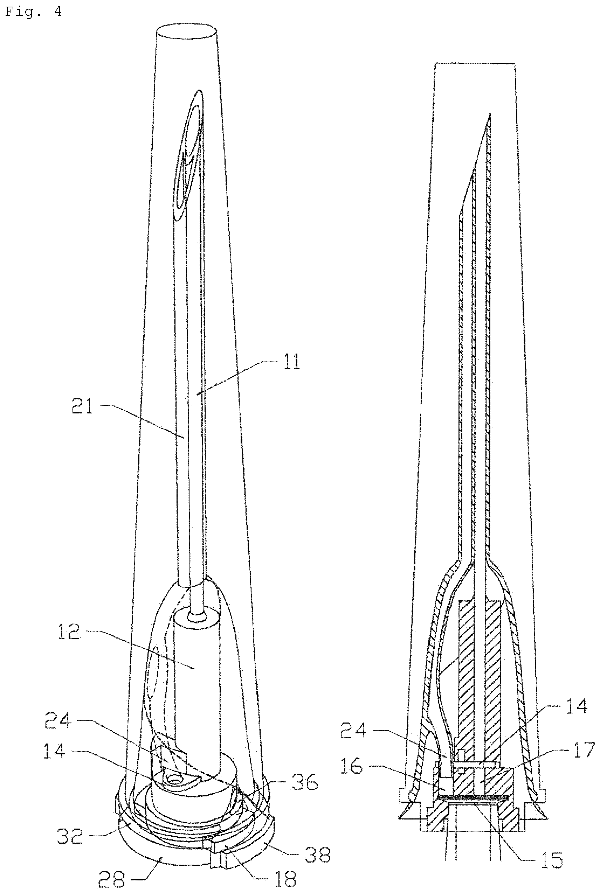

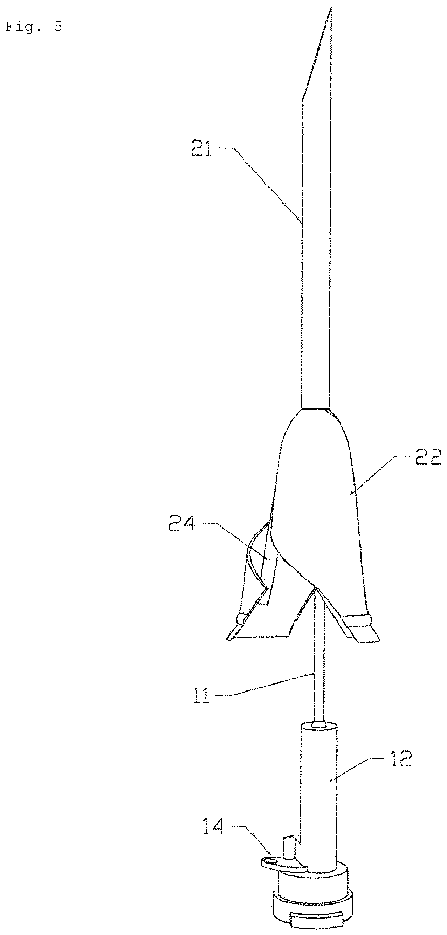

Next, coupling structures and operations of components will be described with reference to FIGS. 4 and 5.

As described above, when the cap 30 is removed, a portion of a lateral surface of the suction part 20 may be cut previously.

To this end, the cap 30 may further include a cutting blade 36 configured to cut a portion of the cut groove 26 while the cap 30 is separated from the suction part 20. An initial position of the cutting blade 36 corresponds to a start point of the cut groove 26.

According to the above-described preliminary configuration, in addition to the removal of the cap 30, a side surface of the suction part 20 that may be easily cut with a small force is longitudinally cut and expanded (widened) by a rotating and removing operation, in other words, a rotating and cutting operation, of a user, and is separated toward the upper side of the needle 11. Accordingly, the entire needle part 10 is exposed and is then removed. To this end, the cut groove 26 is formed on a side surface of the suction part 20, and the cut groove 26 preferably extends in a helical shape to almost a distal end surrounding the needle 11 throughout the entire extension 22.

Although the total number of pitches of the helical cut groove (the number of twists) is illustrated as one in the embodiments of the drawings, the number of pitches may be two or three as needed. As the cut groove is twisted more, a transverse rotational movement of the extension 22 becomes stronger. Thus, although it is advantageous for the connection tube 24 to surely rotate the rotating plate cap 14, a finger may come into contact with the needle part 10 to increase a risk of pollution by a degree of twist, and thus, the user compromises on an appropriate degree of twist.

Meanwhile, a space in which the needle 11 is accommodated and the intake tube 21 configured to suction the injection liquid are separated from each other. Anyway, the intake tube 21 to be separated to the upper side needle 11 does not need to be cut as long as the intake tube 21 may rotate about the needle 11, and thus, the cut groove is not required.

However, it is preferable that a distal end of the suction part 20, exactly, a distal end of the intake tube 21, has a needle shape having a section thickness (hardness is secured to some extent) to pierce an ampoule cap formed by a rubber membrane, as needed.

The soft connection tube 24 connecting the intake tube 21 and the filter 15 to each other between the intake tube 21 and the filter 15 is further arranged on an inner peripheral surface of the suction part 20, and the connection tube 24 is inserted into the inlet hole 16 provided in the support 12 below the needle part. In an operation process, a distal end of the connection tube may be partially formed of high elastic silicone for a human body (used as a prosthetic material) to improve sealing ability. Of course, for mass production, the entire suction part including the intake tube 21, the extension 22, and the connection tube 24 may be manufactured through one time of injection molding using one material.

The needle part 10 operated in conjunction with the suction part 20 will be described in detail.

Main configurations of the needle part 10 are focused on the support 12 supporting the needle 11 inserted into an upper end of the needle part 10. The rotating plate accommodating part 13 and the outlet hole 17 may be further formed inside the support 12, and the inlet hole 16 and a needle part handle 18 may be formed in a lower root of the support.

For reference, although the support 12 is indicated based on the drawings in one embodiment, the support itself is conceptually included in the needle part. The geometric features of the needle part 20 which corresponds to a comprehensive concept are not determined by whether the support exists or not and the geometric features of the support.

Meanwhile, the rotating plate cap 14, which is a component assembled and coupled to the needle part as a part of the needle part, is a core component which is rotatably inserted into and arranged in the rotating plate accommodating part 13 and is interlocked with the suction part 20. Further, the rotating plate cap 14 is manufactured of soft polymer resin or silicone having more enhanced elasticity.

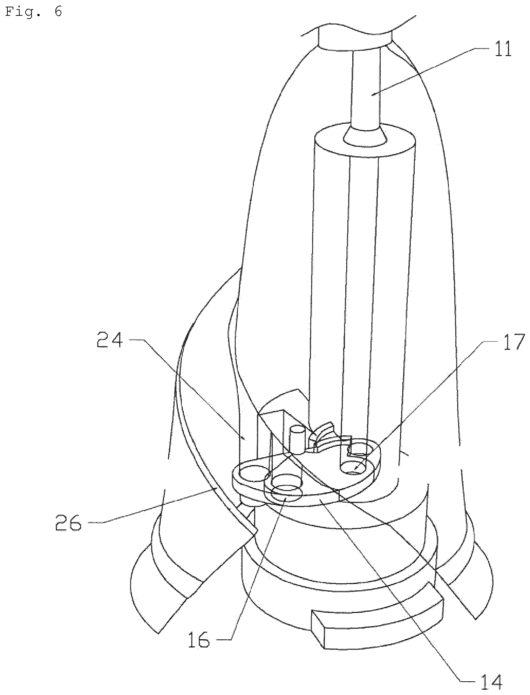

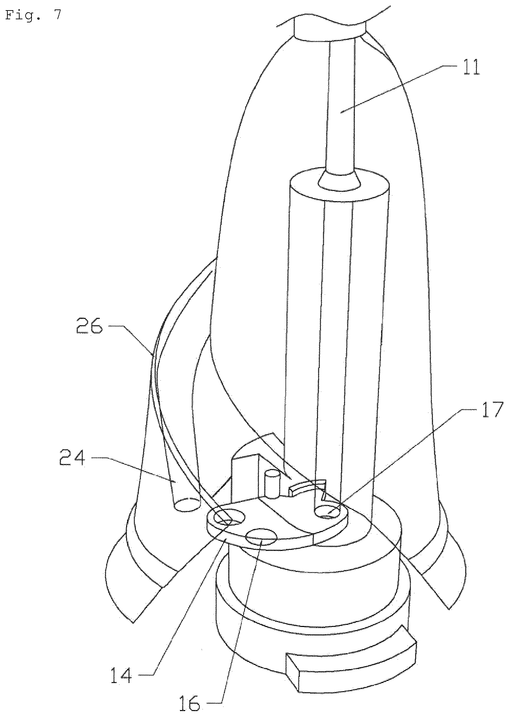

FIGS. 6 and 7 are views separately illustrating a main operation structure of the filtering needle. A process of coupling, moving, and separating operation components will be described with reference to the drawings. Although the drawings have many unrealistically enlarged portions to assist in the structural understanding, those skilled in the art may receive help when performing design and application. Further, it is noted in advance that the portions unrealistically illustrated with ignoring dimensions are not intended to affect the enablement and the scope of the present invention.

When viewed from the above, the suction part handle 28 is arranged in a longitudinal direction (Y-axis direction) of a portion where the cut groove 26 is formed. Here, the needle part handle 18 and a cap handle 38 are arranged in a direction (X-axis direction) that is perpendicular to the longitudinal direction so as not to overlap each other.

That is, the user holds the suction part handle 28 with one hand and pulls the cap handle 38 with the other hand to remove the cap 30, and then holds and rotates the suction part handle 28 while gripping the syringe cylinder, to pull the suction part 20 over the needle part 10 while removing the suction part 20 along the cut groove 26. In this process, the hands do not come in contact with the needle part 11, so that pollution is fundamentally prevented.

The rotating plate cap 14 is connected to the connection tube 24 to close the filter 15 or the inlet hole 16 communicating with the filter in conjunction with a cutting and rotating movement of the suction part 20, and to open the outlet hole 17 communicating with the needle 11.

At this time, according to a movement of the suction part, that is, an expanding and separating movement range of the connection tube, the rotating plate cap 14 may be disconnected from the connection tube 24 after closing the inlet hole 16 or opening the outlet hole 17.

Accordingly, an injection liquid in an ampoule flows from the intake tube 21 via the connection tube 24 and the inlet hole 16 to the filter 15, is filtered by the filter 15, and is introduced into the syringe cylinder.

Next, the rotating plate cap 14 closes the inlet hole 16 and opens the outlet hole 17, so that the injection liquid may be injected into a human body through the needle 11.

At this time, the filter 15 should necessarily be arranged below the inlet hole 16, and may be enlarged and arranged to the lower side of the outlet hole 17 as needed. To arrange the filter on a side of the outlet hole 17 is a matter that may be selected in the corresponding technical field as needed, and the arrangement does not affect the inherent technical spirit of the present invention.

Finally, the separated suction part is inserted into the cap 30, and in this state, the cap 30 is fitted in the needle part 10, so that a completely used filtering needle or filtering syringe may be simply and safely discarded.

By the simple and precise operations of the soft components as well as the rotating plate cap 14, the needle 11 is not blocked by a unidirectional valve enabling only outflow, and maintains an opened tube state in which both inflow and outflow are possible. Thus, the filtering needle may perform, without any problems, a function of inserting the needle into a human body, and then pulling a plunger slightly to identify whether blood is detected, which is like a general needle, and glass fragments filtered by the filter 15 are completely isolated from the needle.

The technical spirit of the present invention has been described above through the detailed embodiments. In addition, although there are simple changes or simple expansion examples not included in the present embodiment, the technical spirit of the present invention should be interpreted based on not the technical scope of the embodiments but the appended claims.

**<Description of reference numerals>**

| | |
|---|---|
| 10: Needle part | |
| 11: Needle | 12: Support |
| 13: Rotating plate accommodating unit | 14: Rotating plate cap |
| 15: Filter | 16: Inlet hole |
| 17: Outlet hole | 18: Needle part handle |
| 20: Suction part | |
| 21: Intake tube | 22: Suction part side surface |
| 24: Connection tube | 26: Cut groove |
| 28: Suction part handle | |
| 30: Cap | |
| 32: Cap fixing edge | |
| 36: Cutting blade | 38: Cap handle |

## Claims

1. A filtering needle comprising:
a needle part having an upper portion to which a needle is fixed, a lower portion in which a filter is arranged, and a middle portion to which a rotating plate cap is coupled;
a suction part formed to surround a circumference of the needle part, and configured to supply an injection liquid introduced through an upper intake tube to the filter,
wherein the rotating plate cap closes the filter or an inlet hole communicating with the filter in conjunction with a cutting and rotating movement of the suction part.

2. The filtering needle of claim 1, wherein a cut groove is further formed on a side surface of the suction part, and
wherein while a side surface of the suction part is expanded by a rotating and cutting movement, the entire needle part is exposed, and the suction part is then removed.

3. The filtering needle of claim 2, wherein a cap is fitted and coupled to the suction part, and
wherein a cutting blade configured to partially cut the cut groove while the cap is separated from the suction part is further provided in the cap.

4. The filtering needle of claim 2 or 3, wherein a soft connection tube connecting the intake tube and the filter to each other between the intake tube and the filter is further arranged on an inner peripheral surface of the suction part, and
wherein the rotating plate cap is mechanically connected to the connection tube to interlock with the suction part.

5. The filtering needle of claim 4, wherein a rotating plate accommodating part and an outlet hole are further formed in the needle part, and
wherein the rotating plate cap is inserted into and arranged in the rotating plate accommodating part to be rotationally moved, and opens the outlet hole 17 while interlocking with the suction part.

6. The filtering needle of claim 5, wherein the rotating plate cap closes the outlet hole or opens the outlet hole, and is then uncoupled from the connection tube or a cap hook mechanically connected to the connection tube.

7. A filtering syringe on which the filter needle of any one of claims 1 to 3 is detachably or fixedly mounted.
